(19) Europäisches Patentamt
European Patent Office
Office européen des brevets

(11) Publication number: 0 183 352 A2

# (12) EUROPEAN PATENT APPLICATION

(21) Application number: 85306913.6

(22) Date of filing: 27.09.85

(51) Int. Cl.$^4$: A 61 K 31/185

(30) Priority: 12.10.84 US 660137

(43) Date of publication of application: 04.06.86 Bulletin 86/23

(84) Designated Contracting States: AT BE CH DE FR GB IT LI LU NL SE

(71) Applicant: THE UNITED STATES OF AMERICA represented by the Secretary U.S. Department of Commerce
National Technical Information Service Office of Government Inventions and Patents 5285 Port Royal Road
Springfield, Virginia 22161(US)

(72) Inventor: Yarchoan, Robert
3535 Chevy Chase Lk Dr.
No. 103, Chevy Chase, MD 20815(US)

(72) Inventor: Broder, Samuel
6004 Rossmore Dr.
Bethesda, MD 20814(US)

(72) Inventor: Gallo, Robert C.
8513 Thornden Terrace
Bethesda, MD 20817(US)

(72) Inventor: Mitsuya, Hiroaki
259 Congressional Lane
Rockville, MD(US)

(72) Inventor: Popovic, Mikulas
No. 3 Pooks Hill Road, 401
Bethesda Maryland 20814(US)

(74) Representative: Daley, Michael John et al,
F.J. CLEVELAND & COMPANY 40/43 Chancery Lane
London, WC2A 1JQ(GB)

## (54) Use of suramin for clinical treatment of infection with any of the members of the family of human-t-cell leukemia (htvl) viruses including lymphadenopathy virus (lav).

(57) The present invention is a method for clinical or therapeutic treatment for the relief of illnesses caused by human T cell leukemia virus (HTLV) such as acquired immune deficiency syndrome (AIDS), pre-AIDS (lymphadenopathy syndrome), Kaposi's sarcoma, and human T cell leukemia-lymphoma by the administration of Suramin, preferably by the intravenous administration of Suramin. A concentration of Suramin of 50 μ g/ml or greater has been found effective *in vitro* and a therapeutic range of 50–340 μ g/ml is clinically attainable. A preferred course of intravenous treatment for about six weeks of Suramin giving .2 g at 0 day followed by 1 gram at Days 3, 7, 14, 21, 28 and 35 is utilized.

EP 0 183 352 A2

# USE OF SURAMIN FOR CLINICAL TREATMENT OF INFECTION WITH ANY OF THE MEMBERS OF THE FAMILY OF HUMAN T CELL LEUKEMIA (HTLV) VIRUSES INCLUDING LYMPHADENOPATHY VIRUS (LAV)

The present invention is a method for clinical or therapeutic treatment for the relief of illnesses caused by human T cell leukemia virus (HTLV) such as acquired immune deficiency syndrome (AIDS), pre-AIDS (lymphadenopathy syndrome), Kaposi's sarcoma, and human T cell leukemia-lymphoma by the administration of Suramin, preferably by the intravenous administration of Suramin. A concentration of Suramin of 50 μg/ml or greater has been found effective in vitro and a therapeutic range of 50-340 μg/ml is clinically attainable. A preferred course of intravenous treatment for about six weeks of Suramin giving .2 g at 0 day followed by 1 gram at Days 3, 7, 14, 21, 28 and 35 is utilized.

## Material Information Disclosure

Popovic et al, "Detection, Isolation, and Continuous Production of Cytopathic Retroviruses (HTLV-III) from Patients with AIDS and Pre-AIDS," Science, 224:497, May 4, 1984.

Schupbach et al, "Serological Analysis of a Subgroup of Human T-Lymphotropic Retroviruses (HTLV-III) Associated with AIDS," Science, 224:503, May 4, 1984.

Sarngadharan et al, "Antibodies Reactive with Human T-Lymphotropic Retroviruses (HTLV-III) in the Serum of Patients with AIDS," Science, 224:506, May 4, 1984.

Schupbach et al, "Antigens on HTLV-Infected Cells Recognized by Leukemia and AIDS Sera Are Related to HTLV Viral Glycoprotein," Science, 224:607-610, May 11, 1984.

Mitsuya et al, "Suramin Protects T Cells In Vitro Against Infectivity and Cytopathic Effect of HTLV-III," In Press.

## Description of the Figures

Figure 1 shows the inhibition of $HTLV\text{-}III_B$

infectivity in H9 cells by Suramin and is more fully described in Example 1.

Figure 2 shows the inhibition of cytopathic effect exerted by HTLV-$III_B$-bearing H9 cells against a normal helper/inducer T cell clone (YTA1) by Suramin and is more fully described in Example 2.

## The Invention

This invention is specially in the use of the drug Suramin to treat (1) patients with diseases related to and believed to be caused by the human T cell leukemia family of viruses, including but not limited to AIDS, pre-AIDS (lymphadenopathy syndrome), Kaposi's sarcoma, and human T-cell leukemia-lymphoma; (2) persons who are or have been infected with one or more of these viruses (including seropositive persons) with the aim of preventing the development of disease; and (3) persons who have been exposed to one of these viruses with the aim of preventing HTLV-related diseases.

It is believed and is shown that Suramin can inhibit the replication of HTLV-III in a T cell line and interferes with the reverse transcriptase of HTLV-III. This drug has been used previously for the treatment of onchoceriasis and certain forms of Trypanosomiasis. The use disclosed here, however, is unrelated to its use for the treatment of those parasitic diseases and it is specially a function of this invention to develop a minimal effective range which has been found to be 50 μg/ml or greater.

## Precis of Protocol

Suramin discussed above is a drug which has been used in human beings for many decades. It has been used primarily to treat African sleeping sickness (trypanosomiasis) and onchocerciasis. Its mechanism of action against these very different organisms is still obscure. It has recently been discovered that this drug is an inhibitor of reverse transcriptase, an enzyme

necessary for the replication of retroviruses. In May 1984, Dr. Robert Gallo and his colleagues discovered that Acquired Immunodeficiency Disease Syndrome (AIDS) was caused by a retrovirus denoted by the initials HTLV-III (cf. Science, 224:497 and 506 of 4 May 1984 and 607 of 11 May 1984). It is believed that AIDS is caused and perpetuated by the replication of HTLV-III once a susceptible patient becomes infected with this virus. It has recently been discovered that the *in vitro* replication of HTLV-III can be inhibited by concentrations of Suramin that can be achieved in human beings. This invention therefore notes that an established intravenous regimen of Suramin can inhibit *in vivo* replication of HTLV-III in a patient with AIDS provided that the concentration of Suramin is monitored to 50 μg/ml or greater and that the drug can be safely administered to patients with AIDS.

## The Treating Agent

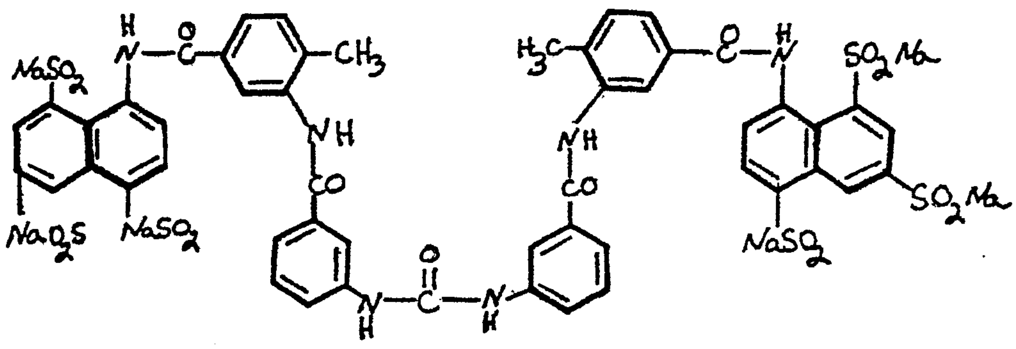

Suramin is a pinkish white flocculant powder, with high solubility in water (more than 10% w/v); its solutions are stable to boiling. Suramin is hygroscopic and absorbs moisture from the atmosphere unless kept in a desiccator; the presence of this unsuspected water may cause error in quantitative experiments. It should be stored in the dark and under dry conditions. In Merck Index (Tenth Edition) Suramin is described as 8,8'-[carbonylbis[imino3,1-phenylenecarbonylimino(4-methyl-3,1-phenylene)-carbonyl-imino]]bis-1,3,5-naphthalene trisul-

fonic acid hexasodium salt; hexasodium sym-bis-(m-aminobenzoyl-m-amino-p-methylbenozyl-1-naphthylamino-4,6,8-tri-sulfonate) carbamide; Bayer 205; 309F, Antrypol; Germanin; Moranyl; Naganol; Nagamin; Naphuride Sodium. $C_{51}H_{34}N_6Na_6O_{23}S_6$; mol. wt. 1429.21. C 42.86%, H 2.40%, N 5.88%, Na 9.65%, O 25.75%, S 13.46%.

Background of AIDS and the Therapeutic Use of Suramin in Connection with the Illness

Of the HTLV-I, -II, or -III related diseases, AIDS is most prominantly related to this invention.

In 1980-81 the apparently new and unexplained disorder called AIDS was recognized. The disorder is a pandemic immunosuppressive disease that predisposes to life-threatening infections with opportunistic organisms and certain neoplasms (especially Kaposi's sarcoma and occasionally lymphoma), which is believed to be indicia of the underlying immune impairment. Characteristically, AIDS is associated with a progressive depletion of T cells, especially the helper-inducer subset bearing the OKT4 surface marker.

Most cases of AIDS have occurred in patients belonging to one of four categories that are referred to as risk groups for epidemiologic purposes: sexually active homosexual or bisexual men (roughly 3/4 of cases); intravenous drug abusers (about 17%); Haitian entrants into the USA (about 5%); and individuals with hemophilia (< 1%). AIDS has developed in patients who have received blood products. Cases of apparent transplacental transmission have also been reported. Moreover, in certain parts of the world, such as Central Africa, the disease may be occurring in individuals who do not belong to one of the known AIDS risk groups.

The expected survival for AIDS patients is less than two years after the diagnosis. A subset of patients with AIDS may develop Kaposi's sarcoma as the only initial manifestation of their disease; and some patients

in this subset have comparatively normal immune function. Nevertheless, a progressive deterioration of immune function is the rule. In those patients in whom Kaposi's sarcoma is life-threatening in its own right, there may be a role for combination chemotherapy or certain interferon preparations. However, no therapy has been shown to reverse the clinical immune impairment, and at present AIDS is considered to be a uniformly fatal disease.

In the Spring of 1984 converging lines of investigation linked a cytopathic member of the human T cell leukemia virus family to the pathogenesis of AIDS. The retrovirus which is now thought to play a role in the etiology of AIDS patients is referred to as HTLV-III (sometimes also called human T-lymphotropic virus), and the data implicating this virus in the causation of AIDS were collected in the Laboratory of Tumor Cell Biology, DCT, NCI (National Institutes of Health, Bethesda, Maryland) by Dr. Gallo and his colleagues. There is strong data to suggest that patients with AIDS or precursor forms of AIDS harbor replicating virions of HTLV-III and that this virus preferentially infects--and then destroys--OKT4 (helper/inducer) T cells. A high rate of replication and detectable viremia characterize the HTLV-III infection seen in AIDS at least early in the disease.

The discovery of a retrovirus linked to the causation of AIDS offers the clinician new strategies for the therapy of AIDS. All retroviruses (including HTLV-III) require a unique enzyme called reverse transcriptase in their natural cycle of replication. Therefore, drugs which inhibit reverse transcriptase are candidates as experimental agents to block HTLV-III replication. In 1979, De Clercq reported in Cancer Letters, 8:9-22 (1979) that Suramin, a well-known anti-trypanosomal agent, was a potent competitive inhibitor of the reverse transcriptase of animal retroviruses. Since the first paper (by Gallo and his colleagues) describing a human retrovirus was

published in 1980, the recent discovery of HTLV-III has re-awakened interest in this agent (as well as other agents known to inhibit reverse transcriptase).

Recent studies connected with this invention have shown that the replication of HTLV-III in vitro can be essentially completely inhibited at Suramin concentrations of 50 μg/ml or greater, a dose which is achievable in human beings according to the published experience with this drug.

Therapeutic Background of Suramin

Suramin is the sodium salt of 8,8'(carbonylbis(imino-3,1-phenylenecarbonylimino(4-methyl-3,1-phenylene)carbonylimino))bis-1,3,5-naphthalenetrisulfonic acid (see formula supra). Suramin was introduced by Bayer (DR) in 1920 as a cure for African sleeping sickness. The drug was found to be very valuable for the therapy of human trypanosomiasis, although its mechanism of action is obscure. During recent years, it has been supplanted by newer agents for this disease. In the 1940's it was discovered that Suramin also acted on onchocerciasis (although trypanosomes and onchocercal worms are very different organisms), and it is available on a limited basis in the United States for the therapy of this disease. For the adult worms of O. volvulus, Suramin may be the only effective compound available to physicians, and it is considered to have an acceptable safety record if administered with careful supervision. It has been given to 27,000 cases in Venezuela without any deaths or serious ill effects. Suramin has also been used in the treatment of non-parasitic diseases. For example, during the 1930's this drug was used to treat pemphigus, frequently in very high doses (e.g., 10 gm in 19 days).

Absorption and Administration

Suramin is given by slow i.v. administration as a 10% aqueous solution made up by adding 10 ml to a 1-

gram vial of dry powder. The drug is poorly absorbed by the oral route. When given by subcutaneous or intramuscular injection, it causes intense local irritation, and suitable precautions are necessary to avoid leakage when the drug is given intravenously.

After intravenous injection, Suramin combines with serum proteins and much of it circulates in the blood. It persists in the bloodstream for very long periods and its excretion in the urine is very slow. When repeated doses of 1 gram are given at 4- or 7-day intervals (as in the therapy of trypanosomiasis), there is considerable accumulation in the plasma and concentrations as high as 340 micrograms/ml may be reached.

Recognized Toxicity in Humans From Suramin

An understanding of the toxicity of this agent derives almost exclusively from its use in treating trypanosomiasis and onchocerciasis. It should be recognized that most of the available information comes from its use under very difficult field conditions. Patients who need this drug are sometimes debilitated from other causes and living on a starvation diet. In addition, some of the side-effects described may be due to the release of toxic products by dead or dying worms.

Immediate reactions

(1) Nausea and vomiting

(2) Sudden collapse with nausea, vomiting, diaphoresis, shock, and coma. Collapse may be preceded by a brief period of neuromuscular irritability and congestion of the face and body. One authority (Apted) estimated that this complication occurs in 1/2000 to 1/4500 cases. Patients likely to exhibit this reaction can be detected by administering a test dose (200 mg) first.

(3) Acute abdominal pain occurs rarely.

(4) Urticarial eruptions.

Late reactions (3-24 hours)

(1) Fever

(2) Photophobia and lachrymation, sometimes with palpebral edema.

(3) Abdominal distension and constipation.

(4) Cutaneous hyperesthesias of the soles and palms. The pain begins 24 hours after injection and may persist for a week or longer. The affected skin may desquamate.

Delayed reactions (after some days)

(1) Renal damage is the commonest toxic reaction.

(2) Exfoliative dermatitis. In Venezuela this was observed in approximately 1/1000 cases.

## Outline of Therapeutic Parameters Developed and Utilized With This Invention

(1) Concentration of Suramin in the blood. 50 μg/ml or greater was found to be effective and a range of 50 to 340 μg/ml is clinically achievable in the blood. The presence of a virus of HTLV, presumably HTLV-III, may be tested *in vitro* by a number of methods including immunofluorescence. See below, Example 1. The p24 antigen of HTLV-III is a fraction of HTLV-III. It is separated and used for assay purposes; see Popovic, et al, *Science*, 224:503, May 4, 1984.

(2) Administration i.v. by slow infusion of the dosage of Suramin. The present preferred regimen embodies a trial dosage at 0 days (.2 mg) followed by 4- to 7-day increments of 1.0 grams at Days 3, 7, 14, 21, 28 and 35, totaling 6.2 grams of Suramin in aqueous sterile, which is obtained by dissolving 1 g of solid Suramin in 10 ml of sterile water.

(3) Diseases treated. Although AIDS, a disease stemming from HTLV-IIIb is the primary target, other related diseases such as pre-AIDS (lymphadenopathy syndrome), Kaposi's sarcoma and human T cell leukemia

lymphoma are included within the scope of the invention.

As exemplary for purposes of the present invention, an inhibition of in vivo viral replication is defined as a cessation of HTLV-III replication in the patient with HTLV-III antigenemia taken to signify in vivo replication.

In the present invention, a number of methods may be used to monitor the effectiveness of the drug in patients in addition to following their clinical course. These include following the ratio of T4/T8 lymphocytes, the ability to isolate live virus at various times, measuring for the presence of viral antigens, and other tests for the purposes of replicating virus.

AIDS is an insidious and chronic disease, and especially early in the disease, clinical parameters often fluctuate. In the first group of patients tested with the drug, the clinical course is being monitored and, in addition, specimens are being frozen for testing and assay.

In this in vivo invention is set out the finding that Suramin can block the in vivo infectivity of HTLV-III (in the form of cell-free virions) when assayed in the H9 target population, a line which expresses mature T-cell markers but is permissive for HTLV-III replication and only partially susceptible to its cytopathic effect. Also, the drug blocks the cytopathic effect of HTLV-III against a normal $OKT4^+$ (helper/inducer) T cell clone co-cultured with HTLV-III-bearing H9 cells at concentrations of 50 μg/ml ($3.5x10^{-5}$ mol) or greater, levels that are clinically attainable in human beings.

Example 1

In this in vitro example (see Fig. 1), there was used clone H9 cells to investigate the inhibitory effect of Suramin on the infectivity of the HTLV-III. H9 cells are permissive for the replication of HTLV-III. When the target H9 cells were exposed to the HTLV-IIIB

isolate (in the form of cell-free virions) and cultured for 4 to 6 days in the absence of Suramin, 72-76% of the target H9 cell population became infected and began to produce the virus as determined by an indirect immunofluorescence assay in which rabbit antibodies were used to detect expression of the HTLV-$III_B$ p24 gag protein. A modest, but temporary, protective effect was observed when the H9 cells were cultured in the presence of Suramin at a concentration of 10 μg/ml. However, at concentrations of 100 μg/ml and 1,000 μg/ml, a striking protective effect was observed throughout the six-day interval of culture. In addition, at these higher doses, the H9 population did not develop multiple-nucleated giant cells with ring formation. The latter is a characteristic feature of HTLV-III infection in these target cells. In connection with the results above and that shown in Figure 2, it is believed and shown that the low effective dosage of Suramin *in vitro* is 50 g/ml.

In determining whether Suramin could block the cytopathic effect of HTLV-$III_B$ against a normal helper/inducer T cell growth factor (TCGF)-dependent T cell clone, co-cultured with H9 cells producing HTLV-$III_B$, a known normal T cell clone (YTA1) was used which displayed the following surface membrane phenotype: OKT3+, OKT4+, HLA-DR+, Tac-antigen+, and OKT8-. In addition, YTA1 produced substantial quantities of TCGF and underwent a proliferative reaction in response to antigen in the presence of appropriate accessory cells. Moreover, YTA1 showed helper activity for immunoglobulin production by normal B-cells (Matsushita et al, unpublished).

Infection of H9 cells by HTLV-$III_B$ was performed as follows: The target H9 cells were preexposed to 10 ( ), 100 ( ), and 1,000 ( ) μg/ml of Suramin for two hours, then to polybrene (2 μg/ml) for 30 minutes before HTLV-$III_B$ infection. Control H9 cells ( ) were treated in a similar fashion, except they were not exposed to the drug. Then the H9 cells were centrifuged

(800g) and exposed to HTLV-$III_B$ virus (0.5 ml containing $7.5x10^7$ viral particles) for 45 minutes and resuspended in fresh culture media (RPMI 1640 supplemented with 20% heat-inactivated fetal calf serum, 4 mM l-glutamine, 50 U/ml penicillin, and 50 µg/ml streptomycin), and cultured in flasks at 37°C in 5% $CO_2$-containing humidified air. The cells were continuously exposed to each concentration of Suramin. On days 4, 5, and 6 in culture, the percentage of the target H9 cells containing p24 gag protein of HTLV-$III_B$ was determined by indirect immunofluorescence microscopy as described by Popovic et al, Science 224:497 (1984). Cells were washed with phosphate-buffered saline (PBS) and resuspended in the same buffer at a concentration of $10^6$ cells per milliliter. Approximately 50 µl of cell suspension were placed on a slide, air-dried, and fixed in acetone for 10 minutes at room temperature. Slides were stored at -20°C until use. Twenty microliters of rabbit antiserum to the p24 gag protein of HTLV-III (diluted 1 : 2,000 in PBS) were applied to these preparations and incubated for 50 minutes at 37°C. Then fluorescein-conjugated goat antiserum to rabbit IgG (Cappel Lab., Cochranville, PA) was diluted and applied to the fixed cells for 30 minutes at room temperature. Slides were then washed extensively before microscopic examination under ultraviolet illumination (Nikon Inc., Japan; HBO 100w; filter 450nm NCB10, magnification x320).

Example 2

The results of the experiment to determine the protective effect of Suramin on the survival and growth of a clone normal T cell population exposed to HTLV-$III_B$ in vitro are shown in Figure 2. The YTA1 target cells were pre-exposed to various doses of Suramin for 12 hours and then co-cultured with irradiated (10,000 rad) HTLV-$III_B$-bearing H9 cells, with the dose of Suramin kept constant during the entire time in culture. Uninfected, irradiated H9 cells were added under the same conditions

as a control. All cultures contained 15% (v/v) lectin-free TCGF. The total numbers of viable YTA1 cells as a function of time in culture were determined. In the absence of Suramin, the HTLV-$III_B$-producing H9 cells had exerted a substantial cytopathic effect on the YTA1 population by Day 6 in culture and by Day 10, the YTA1 population was almost completely killed. The addition of Suramin at concentrations of 50 µg/ml or greater enable the YTA1 target population to survive and grow in the presence of HTLV-$III_B$ (at concentrations of 100 µg/ml or higher, the drug clearly blocked the cytopathic effect of the virus but did exert an inhibitory effect on the normal target T cell growth). Thus, Suramin can block the replication of HTLV-$III_B$ in permissive H9 cells and can also inhibit the cytopathic effect of HTLV-$III_B$ upon an $OKT4^+$ T cell clone.

$2x10^5$ YTA1 cells were exposed to various concentrations (0-150 µg/ml) of Suramin for 12 hours in culture tubes (3033 Falcon) in 2 ml of 15% TCGF (vol/vol) (Cellular Products Inc., Buffalo, NY) containing RPMI 1640 supplemented with 15% heat-inactivated fetal calf serum, 4mM l-glutamine, 50 U/ml penicillin, and 50 µg/ml streptomycin at 37°C in 5% $CO_2$-containing humidified air. Then these YTA1 cells were added with the equal number of irradiated (10,000 rad) HTLV-$III_B$-bearing H9 (■) or uninfected H9 (□) cells. Control cells (▒) were cultured without any cells added. Cells were continuously exposed to Suramin and TCGF. The assays were all performed in duplicate. On days 6, 8 and 10 the total numbers of viable cells were counted in a hemocytometer under the microscope by using trypanblue dye exclusion method. When cultured alone in the presence of TCGF, none of irradiated HTLV-$III_B$-bearing H9 or uninfected H9 cells were alive on day 6 in culture. Normal YTA1 cells could be readily distinguished from neoplastic H9 cells by morphology. The YTA1 target cells had been stimulated by soluble tetanus-toxoid antigen plus irradi-

ated (4,000R) autologous accessory cells six days prior to these experiments as part of a cycle of restimulations. Each bar represents the mean number of viable cells $\pm 1$ S.D. of duplicate determinations. The dashed horizontal line depicts the starting number of YTA1 cells.

Most of what is known about the clinical pharmacology of Suramin derives from its use in the therapy of trypanosomiasis and onchocerciasis. After intravenous injection, the drug combines with serum proteins and much of it circulates in the blood. It persists in the bloodstream for very long periods and its excretion in the urine is very slow. The therapeutic regimens in use result in considerable accumulation in the plasma (frequently in excess of 100 μg/ml). Indeed, concentrations as high as 340 μg/ml may be reached under some conditions. These concentrations exceed those found to block the infectivity (Fig. 1) and cytopathic effect (Fig. 2) of HTLV-III.

Therapeutic Response and Results

In the treatment of one increment of patients suffering from AIDS illness they were given the treatment regimen of one to six weeks (or 35 days) administered after a preliminary dose of .2 gram each week by infusion intravenous therapy. Alternate dosages of Suramin from the above may be utilized but the controlling factor would be that a blood level in the patient 50 μ g/ml or greater is achieved. In the initial increment of patients the recognized main toxicities in humans, such as renal allergy, shock and exfoliative dermatitis, did not occur. Additionally, a level of 50 μg/ml or greater in the blood stream was observed. The course of treatment is a long term one.

## CLAIMS

1. The use in the manufacture of a medicament for the treatment of immunodeficiency syndrome (AIDS) of Suramin as an active agent.

2. The use in the manufacture of a medicament for the treatment of the symptoms of HTLV viruses including AIDS, pre-AIDS (lymphadenopathy syndrome), Kaposi's sarcoma and human T-cell leukemia-lymphoma of Suramin as an active agent.

3. The use of Claims 1 or 2 wherein the active agent is in unit dosage form to supply at least 50 µg/ml when administered to an environment of use.

4. The use of any preceding claim wherein the active agent is prepared as injectable solution.

5. The use according to Claim 4 wherein the solution is aqueous and comprises 1 gram of solid active agent and about 10 ml of sterile water.

FIG.1

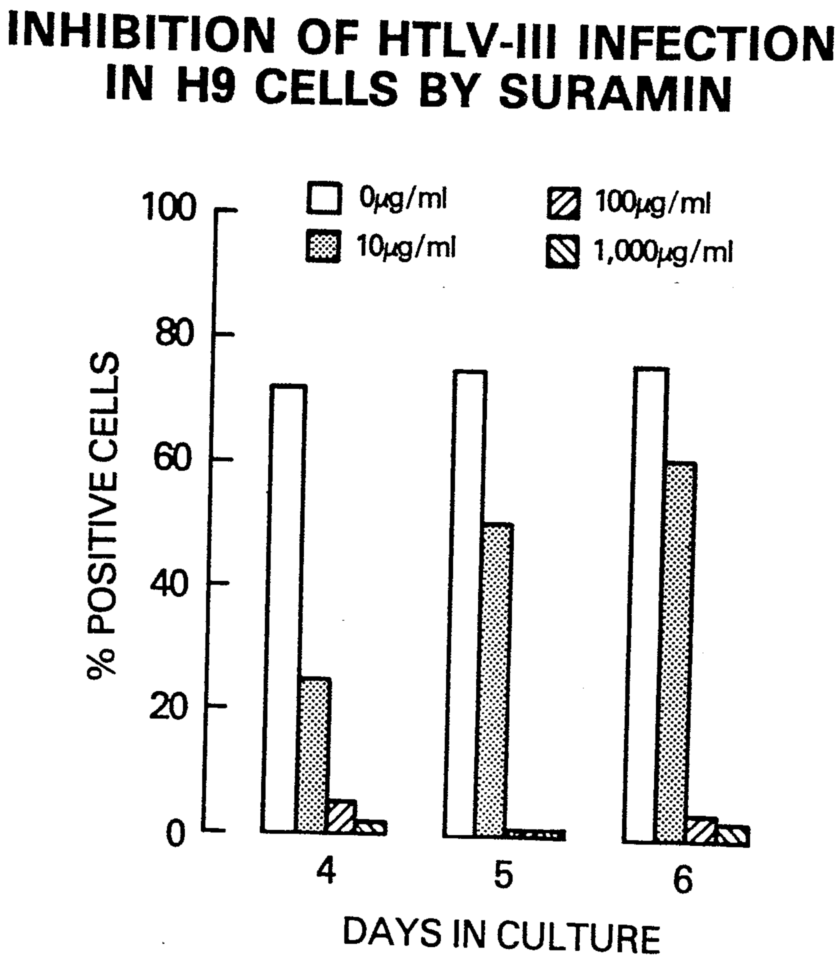
INHIBITION OF HTLV-III INFECTION
IN H9 CELLS BY SURAMIN
0μg/ml
10μg/ml
100μg/ml
1,000μg/ml
100
80
60
40
20
0
% POSITIVE CELLS
4
5
6
DAYS IN CULTURE

FIG.2

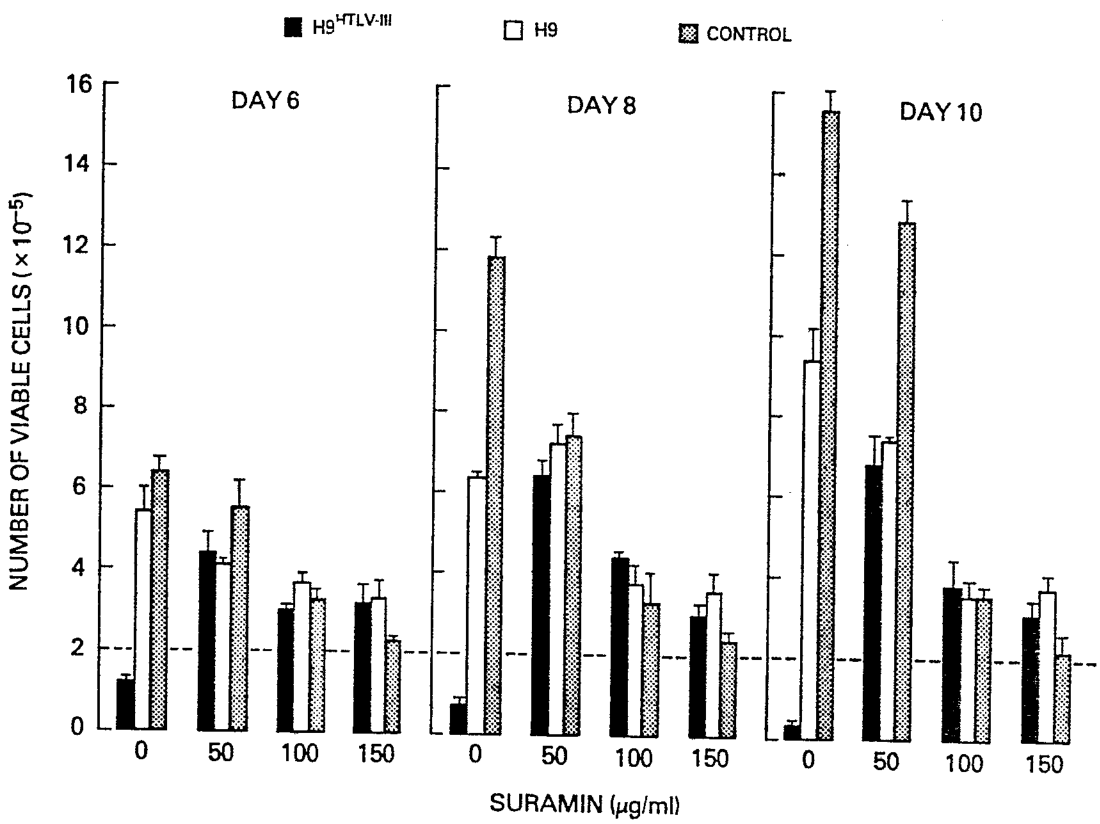
H9 HTLV-III
H9
CONTROL
DAY 6
DAY 8
DAY 10
NUMBER OF VIABLE CELLS (×10⁻⁵)
16
14
12
10
8
6
4
2
0
0
50
100
150
SURAMIN (μg/ml)